# EUROPEAN PATENT SPECIFICATION

(11) **EP 2 957 224 B1**
(45) Date of publication and mention of the grant of the patent: **29.11.2017**
(21) Application number: 15425007.0
(22) Date of filing: 04.02.2015
(51) Int. Cl.: A61B 5/00, A61B 5/11, G06F 19/00

(54) **PORTABLE DIAGNOSTIC SYSTEM FOR MONITORING BIOMEDICAL PARAMETERS**
TRANSPORTABLES DIAGNOSESYSTEM ZUM ÜBERWACHEN VON BIOMEDIZINISCHEN PARAMETERN
SYSTÈME DE DIAGNOSTIC POUR SURVEILLER LES PARAMÈTRES BIOMÉDICAUX

(30) Priority: 17.06.2014 IT VI20140153
(43) Date of publication of application: 23.12.2015
(73) Proprietor: Zulu Medical S.r.l., 31056 Roncade (TV) (IT)
(72) Inventor: Furlanetto, Riccardo, 31056 Roncade (TV) (IT)
(74) Representative: De Tullio, Michele Elio

(56) References cited:
- GB-A- 2 475 091
- GB-A- 2 494 303
- US-B2- 8 079 953

## Description

### Field of application

The present invention is generally applicable to the technical field of medical diagnostic devices and has particularly for object a portable diagnostic system for monitoring biomedical parameters of the human body, particularly suitable to be used by qualified healthcare professional.

### State of the art

It's known the use of diagnostic systems suitable to be used to monitor biomedical parameters of an individual in the post-operative phase or when subjected to an intensive medical therapy.

In particular, it is possible to monitor in real time some vital parameters of the individual such as, for example, pressure and saturation in blood, pulse rate, body temperature, ECG, glycemic index or other similar parameters.

Distinctive characteristic of said systems is to provide a local or remote access of the monitored parameters, allowing qualified healthcare professional to evaluate the health of the individual even remotely.

For example, diagnostic systems of this type are used in intensive care units or cardiology of hospital or medical clinics, to provide real time vital parameters of one or more patients to healthcare professionals located in the same structure or in different ones.

Generally healthcare professionals have a mobile interface device, such as a smartphone or tablet, adapted to display in real time the progress of biomedical parameters detected by the system on one or more individuals.

However, said systems are designed to monitor the health status of an individual being hospitalized at a particular department of a medical facility and which undergoes only small displacements within the same.

In particular, these systems exhibit considerable sizes that make difficult to transfer them on ambulances or other similar vehicles with the consequent drawback that can't be used by medical staff that works in case of emergency.

In addition, said systems work properly only when an individual is substantially motionless while risk to be damaged when the latter undergoes sudden movements, as it happens in emergency situations.

To overcome this drawbacks portable systems have been developed for monitoring biomedical parameters of an individual which shows reduced overall dimensions and can be easily carried in mobile medical units.

For example , from US8079953 is disclosed a portable medical instrumentation, suitable to send to a server a plurality of biomedical parameters associated with an individual who may stay inside a medical facility, in a home environment or in open spaces.

In particular, said system includes a plurality of sensors applicable to the body of the individual to detect certain vital parameters such as, for example, ECG, EMG, EEG, blood pressure, oximetry or other similar parameters.

Analog signals generated by each of said sensors are then converted into digital format and sent to a remote CPU through strandardized communications means such as, for example, an Ethernet or wireless network. Furthermore, this system is also able to provide a GPS geolocation.

Numerical data received from the CPU are appropriately processed to enable storing in a database connected to one or more remote personal computer in order to be viewed by authorized healthcare professionals.

From GB2494303 is known a portable device for the monitoring of biomedical parameters of a patient showing a plurality of sensors adapted to detect the vital parameters of the patient according to the preamble of claim 1.

Said device comprises a communication system configured to send collected data to a central computer and a plurality of movable interface devices of pocket type are also provided to be used by healthcare professionals in which all the vital parameters of the patient are viewable in real time.

In both solutions described the interface devices and medical devices for the detection of biomedical parameters are powered by a rechargeable battery.

A first drawback of this solution is represented by the fact that the life of rechargeable batteries used in such systems is relatively low.

In particular, the limited duration of batteries can be particularly unfavourable healthcare professionals have to work for a long time in outdoor environments without electric connections, as well as when many individuals are monitored at the same time causing a significant increase of electrical consumption due to the same devices.

In addition, a further drawback of said solutions is the fact that devices are kept turned off during the moving of emergency vehicles in order to preserve the charge of their batteries.

This may delay the monitoring of the individual as some sensors may have not negligible start-up times.

It is also necessary to charge individually each interface device and each medical detection device and this may increase the risk that the worker forgets to connect some apparatus to the power grid making it substantially unusable when the emergency occurs.

Furthermore, to maintain the system always efficient it is necessary to check constantly the state of charge of any device and this needs a not neglegibile time and significantly decrease the comfortable use of the same system.

### Presentation of the invention

The aim of the present invention is to overcome the drawbacks evidenced above, by providing a portable diagnostic system for monitoring biomedical parameters of the human providing characteristics of high efficiency and relative cheapness.

A particular aim of this invention is to make available a portable diagnostic system for monitoring biomedical parameters of the human body which has long operating time.

A further aim of this invention is to make available a portable diagnostic system for monitoring biomedical parameters of the human body providing a life and operating time higher than the current diagnostic systems.

A further aim of this invention is to make available a portable diagnostic system for monitoring biomedical parameters of the human body easily powered during its transport to the area of intervention.

A further aim of this invention is to make available a portable diagnostic system for monitoring biomedical parameters of the human body which providing a flexible configuration and particularly limited dimensions, in order to be easily used even in particularly extreme environments.

A further aim of this invention is to make available a portable diagnostic system for monitoring biomedical parameters of the human body allowing to power simultaneously all said electrical devices, reducing risk of unintentionally keep disconnected some of them.

These aims, and furthers that will become apparent hereinafter, are achieved by means of a portable diagnostic system for monitoring biomedical parameters of the human body in accordance with claim 1.

Advantageous embodiments of the invention are obtained according to the dependent claims.

### Brief description of Drawings

Further features and advantages of the invention will become more apparent from the detailed description of some preferred embodiments but not exclusive of a portable diagnostic system for monitoring biomedical parameters of the human body according to the invention, being purely illustrative and therefore not limiting with reference to the accompanying drawings that illustrate, respectively:
FIG.1 is a simplified block diagram of the portable diagnostic system according to the invention;
FIG.2 are side views of a first detail used in the system of Fig. 1;
FIGS.3 AND 4 are simplified block diagrams of the electrical circuit contained within the detail of FIG. 2 ;
FIG.5 reproduces come images displayed in the second detail of the assembly of Fig. 1

### Detailed description of a preferred embodiment

With reference to the figures, it is illustrated a diagnostic system for monitoring biomedical parameters of the human body, generally indicated by the reference number 1, in particular intended to be used by healthcare professionals such as, for example, physicians, nurses and first aid workers.

In particular, said diagnostic system 1 usable both in closed environments, such as, for example, medical clinics, hospital wards or mobile clinics, and in open spaces where emergency situations generally occur with necessity of assisting one or more individuals.

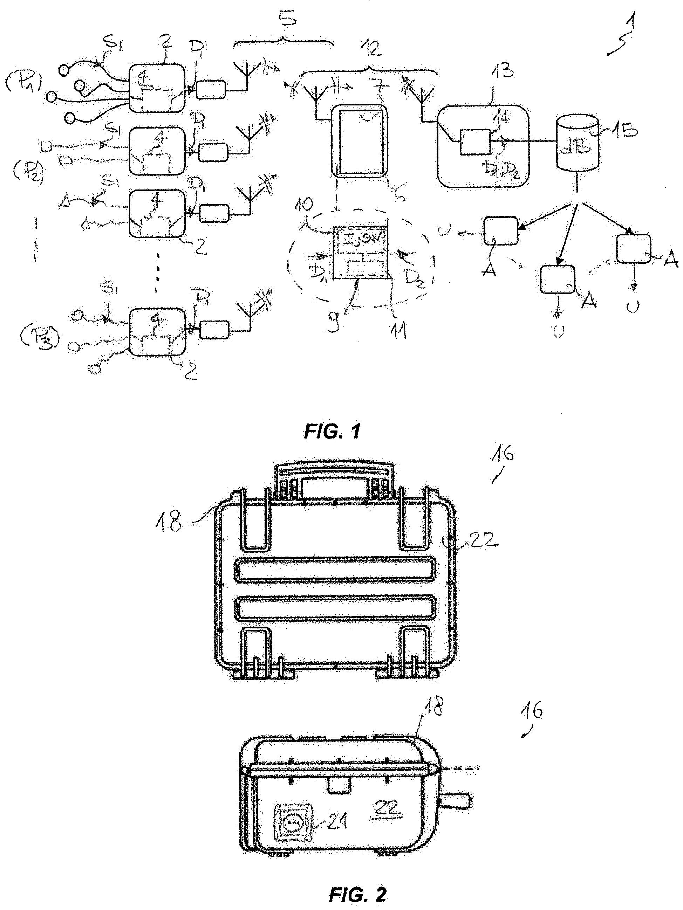

In its basic embodiment, the portable diagnostic system 1 for monitoring the biometric parameters of the human body, showed in FIG.1, comprises at least one diagnostic device 2 having one or more sensors 3 applicable to the human body to generate a first set of data D1 associated to a biomedical parameter P of an individual.

Preferably, the system 1 may include several diagnostic devices 2 equipped with corresponding sensors 3 for detecting vital biomedical parameters P of an individual.

For example, in a preferred configuration of the invention the system 1 may include a plurality of diagnostic devices 2 adapted to detect the following biomedical parameters P of an individual:
- ECG (Electrocardiogram);
- Spirometry;
- Glycemic Index;
- Pulse heart;
- Blood pressure;
- Level of oxygen saturation in the blood;
- Temperature and/or body weight;
- INR (prothrombin time).

Appropriately, each biomedical parameter P may be detected by a single diagnostic device 2 or, alternatively, a given device 2 can be equipped with different type of sensors 3 in order to detect simultaneously several diagnostic parameters P of an individual.

Of course, the above list is only indicative and different configurations of the system 1 may be realized to detect biometric parameters different from those listed.

The sensors 3 of each electronic device may generate an analogic electrical signal S₁ having performance related to the particular biomedical parameter P detected in the human body.

Nevertheless, the diagnostic device 2 may include a converter 4 adapted to convert the analog signal S₁ from sensors 3 in a first series of data D₁.

In the preferred configuration of the invention, the converter 4 will be of the type analog-to-digital to generate a first series of numerical data D₁.

For example, diagnostic devices 3 may provide a serial RS232 or USB (Universal Serial Bus) type output, not showed in the drawings, in order to provide the first series of digital data D₁ by means of standardized communication protocols and known thereof.

The system 1, futhermore, includes first communication means 5 adapted to transmit the first series of data D₁ detected by the diagnostic device 2 to interface means 6 including a display 7.

For example, interface means 6 may be realized by means of portable or pocket electronic device such as smartphones, tablets, laptops or other similar devices.

In addition, both diagnostic devices 2 and interface means 6 may be powered by the rechargeable batteries 8 integrated in the same.

First communication means 5 may include a wired transmission line, of Ethernet type of carrier waves, or more advantageously may take advantage of the mobile telephony architecture networks to realize wireless communication according to the TCP/IP Protocol, as showed schematically in FIG. 1

Furthermore, it is not excluded the opportunity of using first communication means 5 adapted to transmit the first series of data D₁ by means of Bluetooth protocol, and also providing them with a geo-locator GPS device, not showed in the figures.

Said last configuration of the first transmission means 5 can be particularly useful when the system 1 is to be used in unaccessible environments not reached by radio signals of the mobile phone network.

Interface means 6 may comprise first processing means 9 adapted to process the first series of data D₁ in a second series of data D₂ to be displayed on the display 7.

Therefore, also the second series of data D₂ will be of the digital type.

In particular, first processing means 9, may include a storage circuit 10 on which are stored instructions I of a computer software SW, and a processor 11 connected to the storage circuit 10 to process first series of data D₁ in the second series of data D₂.

Instructions I of the computer software SW also allow the healthcare professional to record in the storage circuit 10 a plurality of information associated to the individual being monitored, as well as display in graphical and temporal form the second series of data D₂ which refers to the same individual.

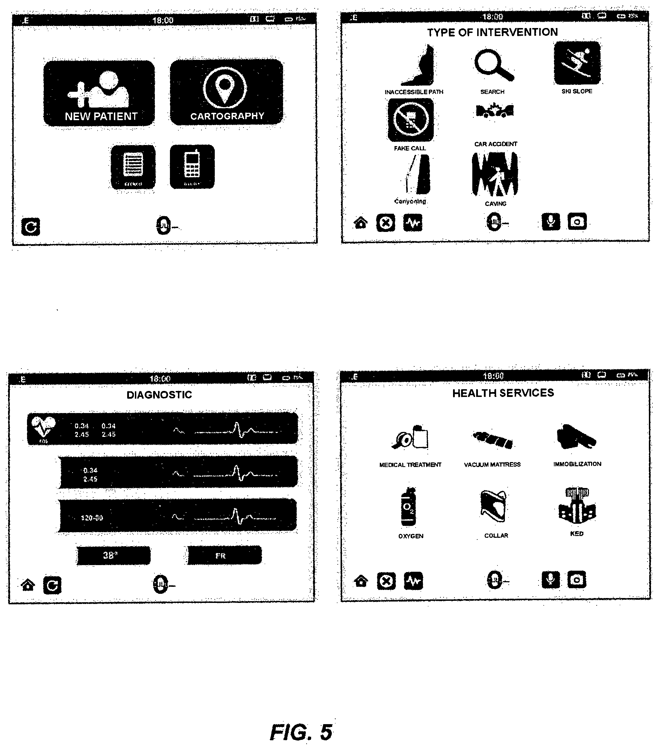

For example, FIG. 5 shows some images displayed on the display 7 of the interface means 6 during the execution of the computer software SW.

As is easily noticed, in the section called "diagnostic" where real time courses of the second series of data D₂ are showed alongside other screens where the healthcare professional can input information or can operate choices among several options provided by the computer software SW.

For example, the healthcare professional can enter data about a new individual or recover those of a person already under monitoring, as showed in the first screen, or he can establish what kind of intervention is going to do on the person, as showed in the second screen called "Type of Intervention", and in addition he can list which health services are performing on the individual as clearly showed in the fourth screen named "Health services".

It is understood that instructions I of the computer software SW stored in the memory storage 10 can also allow to display on the display 7 of interface means 6 more series of second data D₂ associated with different individuals.

Preferably, the system 1 may include second means of communication 12 adapted to transmit the first D₁ and / or the second series of data D₂ from interface means 6 to a central processing unit 13.

As declared for first communication means 5 remains valid also for second communication means 12 which ,therefore, may be preferably selected within the group which comprises digital communication Ethernet means, wireless, Bluetooth or similar.

The central processing unit 13 may be of remote type and may include second processing means 14 adapted to process the first series of data D₁ or the second series of data D₂ in order to store them in a database 15.

The latter may be physically contained in the central processing unit 13 or, according to the current cloud computing architectures, may be stored in a server, not shown, geographically spaced from the central processing unit 13 and connected to the same through internet or a dedicated network.

Advantageously , the access to the first D₁ and/or to the second series of data D₂ stored in the server can be allowed to one or more authorized healthcare users U also located in other places respect to the one where the individual is monitored.

For example, the second series of data D₂ can be displayed in real time on electronic devices A located in the ward of the nearest hospital or in more wards of the same clinic or in other medical auxiliary structures that can take care of the monitored individual.

According to a peculiar aspect of the invention, the system 1 comprises confining means 16 adapted to support diagnostic devices 2 and interface means 6.

Confining means 16 have a power supply circuit 17 connected to the diagnostic device 2 and to interface means 6 to provide continuous power to the same.

In a particular system configuration, means confinement 16 comprise a suitcase 18, showed in FIG. 2 within which are housed diagnostic devices 2 and interface means 6.

The use of a suitcase 18 makes particularly easy transporting diagnostic devices 2 and display means 6 when the system 1 is supplied to first aid staff.

In particular, as shown schematically in FIGS, 3 and 4 , the suitcase 18 may contain within it a tablet 6' for displaying the second series of data D₂ and a plurality of diagnostic devices 2.

In an alternative embodiment of the system, not showed in figures, confining means 16 may comprise a trolley equipped with wheels and adapted to support all of the diagnostic devices 2 and interface means 6.

The choice of a trolley or other similar containing element is particularly suitable when the system 1 is used in the outpatient areas where the physician needs to quickly monitor some biomedical parameters P of a patient undergoing a visit.

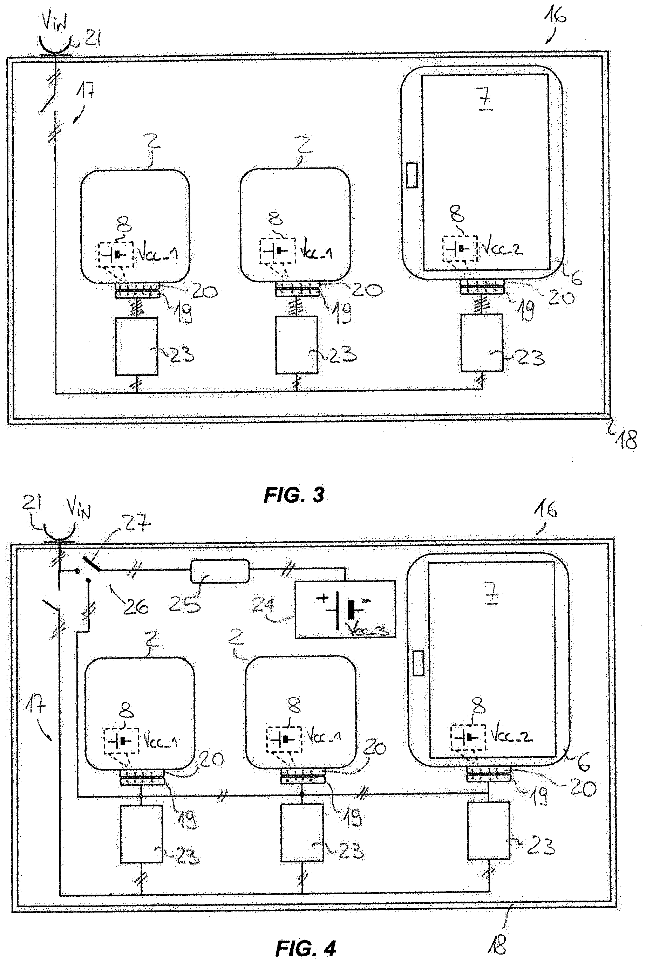

Suitably, as shown schematically in FIGS. 3 and 4, the power supply circuit 17 may include one or more connectors 19 male, or female, respectively coupled to corresponding connectors 20, female, or male, associated with diagnostic device 2 and interface means 6.

In particular, the suitcase 18 or the trolley may include within housing compartments for diagnostic devices and interface means, not shown, and connectors 19 of the supply circuit 17 may be placed in correspondence of said compartments.

In this way, when the apparatus 2, 6 being housing in the corresponding compartment will be possible to couple connectors 19 of the supply circuit 17 with those of diagnostic devices 2 or of interface means 6 ensuring the power supply of the same and/or charging batteries 8.

As shown schematically in FIGS. 2 to 4, power supply circuit 17 may include an electrical socket 21 connectable to an external electric source, not shown in figures.

The socket 21, as suitably shown schematically in FIGS. 2 to 4, may be anchored to the outer surface 22 of the suitcase 18 or of trolley.

By means of the socket 21, it will be possible to promote the electrical connections of connectors 19 of the circuit 17 to the external source thus ensuring power supply of diagnostic devices 2 and /or interface means 6 during their housing in suitcase 18.

In case the emergency vehicle is equipped with an external electrical source, by means of the socket 21 it will be possible to recharge or preserve the charge of accumulators 8 of diagnostic devices 2 and interface means 6 also during the transport of suitcase 18 to the area of intervention.

For example, the socket 21 may be adapted to allow connection of the power supply circuit 17 with a low voltage source or, alternatively, with a civil or industrial driving force of standard type, of about 220V or 380V.

When the power source is sinusoidal and has an effective high value, as in the case of the known driving forces, the power supply circuit 17, may include means of the processing 23, showed in FIGS. 3 and 4, adapted to convert the voltage V_{IN} of the source in the socket 21 to a DC voltage corresponding to the nominal VCC_1 of the diagnostic devices 2 or the nominal VVC_2 of interface means 6.

For example, processing means 23 may include a transformer voltage reducer, not shown, connected to the source and located upstream of a rectifier circuit, also not visible in figures, connected to the connectors 19 of the supply circuit 17.

In a particular variation of the system 1, shown in FIG. 4, power supply circuit 17 may comprise at least one rechargeable battery 24 connected to the connectors 19 and having a nominal output voltage VCC_3 predetermined substantially corresponding to the nominal supply voltage VCC_1; VCC_2 of diagnostic devices 2 and interface means 6.

In this way it will be possible to supply diagnostic devices 2 and interface means 6 by means of the external electrical source connected to the spcket 21 or, alternatively by menas of the rechargeable battery 24.

Preferably, nominal output voltage VCC_3 of the rechargeable battery 24 may be comprised between 6V and 24V and preferably equal to 12V.

As shown schematically in FIG. 4, rechargeable battery 24 may be electrically connected to the socket 21 by means of recharging means 25 adapted to convert the source voltage V_{IN} to a DC voltage equal to the nominal voltage of the rechargeable battery VCC_3.

In a way similar to processing means 23, also charging means 25 may include a transformer voltage reducer and a rectifier circuit, both not visible in figures.

Advantageously, battery 24 can be removably housed inside the suitcase 18 in order to enable a healthcare professional that needs to recharge one or more devices 2, 6 to pull it out from the same to use it as buffer battery nearby the individual.

Preferably, as shown schematically in FIG. 4, power supply circuit 17 may include selections means 26 adapted to selectively promote connection of battery 24 to the socket 21 or connection of battery 24 to diagnostic device 3 and/or to interface means 6.

In particular, selection means 26 may include a selector 27, schematically shown in FIG. 4, controlled by an operator depending on the circumstances.

Furthermore, it may be provided a main switch, not shown in figures, adapted to close/open selectively the power supply circuit 17 in order to provide voltage to diagnostic devices 3 and to interface means 6 only what is necessary to recharge the accumulators 8.

The portable diagnostic system for monitoring biomedical parameters of an individual according to the invention is susceptible of numerous modifications and variations all comprised within the inventive concept expressed in the accompanying claims.

All details may be replaced by other technically equivalent elements and materials may be different according to the requirements, without move away from the scope of the invention.

Although the portable diagnostic system has been described with particular reference to the accompanying figures, reference numbers used in the description and in the claims are used to improve the intelligence of the invention and do not constitute any limitation to the scope of protection which has been claimed.

### Industrial Applicability

The present invention is industrially applicable because it can be realized on an industrial scale by industries belonging to the sector of the production of equipment and diagnostic systems for medical purposes.

## Claims

1. A portable diagnostic system (1) for monitoring biomedical parameters (P) of the human body, the system comprising:
- at least one diagnostic device (2) having one or more sensors (3) applicable to the human body to generate a first series of data (D1) associated with at least one biomedical parameter (P),
- an interface means (6) including a display (7) and a first process means (9), the first process means (9) associated to said interface means (6) configured to process said first series of data (D1) to a second series of data (D2) suitable to be displayed in said display (7),
a first communication means (5) adapted to transmit said first series of data (D1) measured by at least one diagnostic device (2) to said interface means (6),
- a second communication means (12) adapted to transmit said first (D1) and/or said second series of data (D2) from said interface means (6) to a central process unit (13),
- a second process means (14) associated with said process unit (13) to configured to process said first (D1) and/or said second series of data (D2) and store them in a database (15),
- **characterized in that** the portable diagnostic system comprises a confining means (16) designed to support said at least one diagnostic device (2) and said interface means (6),
- an electrical supply circuit (17) provided inside said confining means (16) and adapted to be connected to said at least one diagnostic device (2) and to said interface means (6) for continuously powering the same when being housed in the confining means,
- the electrical supply circuit comprises one or more male connectors (19), respectively female connectors, movably coupled to corresponding female connectors (20), respectively male connectors, associated to said at least one diagnostic device (2) and to said interface means (6),
- the electrical supply circuit comprises an electrical socket (21) connectable to an external electrical mains and electrically connected to one or more connectors (19) of said electrical supply circuit (17), wherein
said electrical supply circuit (17) comprises transformation means (23) adapted to transform the mains voltage (V_{IN}) present in said socket (21) into a DC voltage (V_{CC_1}; V_{CC}_₂) corresponding to the nominal voltage of said at least one diagnostic device (2) and/or of said interface means (6).

2. System as claimed in claim 1, **characterized in that** said supply circuit (17) comprises at least one rechargeable battery (24) connected to said connectors (19) of said electrically supply circuit (17) and having a predetermined nominal DC output voltage (VCC_3) corresponding to the nominal voltage (VCC_1; VCC_2) of said at least one diagnostic device (2) and/or of said interface means (6).

3. System as claimed in claim 2, **characterized in that** said rechargeable battery (24) is connected to said electrical socket (21) by recharging means (25) designed to transform the mains voltage (VIN) present in said socket (21) in a DC voltage corresponding to the nominal voltage (VCC_3) of said rechargeable battery (24).

4. System as claimed in claim 3 **characterized in that** said supply circuit (17) comprises selection means (26) adapted to selectively connect said battery (24) to said electrical socket (21) or said battery (24) to said at least one diagnostic device (2) and/or to said interface means (6).

5. System as claimed in claim 1, **characterized in that** said interface means (6) comprise a storage circuit (10) on which the instructions (I) of a software (SW) are stored and a processor (11) connected to said storage circuit (10) to process said first series of data (D1) according to said instructions (I) and generate said second series of data (D2).

6. System as claimed in claim 1, **characterized in that** said first (D1) and said second series of data (D2) are digital, said first communication means (5) and said second communication means (12) being chosen between the group comprising the Ethernet communication standards, wireless communication standards, Bluetooth or similar communication standards.

7. System as claimed in claim 6, **characterized in that** said at least one diagnostic device (2) comprises a converter (4) adapted to transform the analogic electrical signals (S1) generated by one or more sensors (3) into said first series of digital data (D1).

## Patentansprüche

1. Transportables Diagnosesystem (1) zum Überwachen von biomedizinischen Parametern (P) des menschlichen Körpers, wobei das System Folgendes umfasst:
- mindestens ein Diagnosegerät (2) mit einem oder mehreren Sensoren (3), die am menschlichen Körper angebracht werden können, um eine erste Reihe von Daten (D1) zu erzeugen, die mit mindestens einem biomedizinischen Parameter (P) in Verbindung stehen,
- ein Schnittstellenmittel (6), das eine Anzeige (7) und ein erstes Verarbeitungsmittel (9) umfasst, wobei das mit diesem Schnittstellenmittel (6) verbundene erste Verarbeitungsmittel (9) gestaltet ist, um diese erste Reihe von Daten (D1) zu einer zweiten Reihe von Daten (D2) zu verarbeiten, die geeignet sind, auf der Anzeige (7) angezeigt zu werden, ein erstes Kommunikationsmittel (5), das geeignet ist, die von mindestens einem Diagnosegerät (2) gemessene erste Reihe von Daten (D1) an das Schnittstellenmittel (6) zu übertragen,
- ein zweites Kommunikationsmittel (12), das geeignet ist, die erste (D1) und/oder die zweite Reihe von Daten (D2) von dem Schnittstellenmittel (6) an eine zentrale Verarbeitungseinheit (13) zu übertragen,
- ein zweites Verarbeitungsmittel (14), das mit der Verarbeitungseinheit (13) verbunden und gestaltet ist, die erste (D1) und/oder die zweite Reihe von Daten (D2) zu verarbeiten und sie in einer Datenbank (15) zu speichern,
**dadurch gekennzeichnet,**
- **dass** das transportable Diagnosesystem ein Einschließungsmittel (16) umfasst, das gestaltet ist, um mindestens ein Diagnosegerät (2) und das Schnittstellenmittel (6) zu tragen,
- eine elektrische Versorgungsschaltung (17), die innerhalb des Einschließungsmittels (16) vorgesehen und geeignet ist, mit dem mindestens einen Diagnosegerät (2) und dem Schnittstellenmittel (6) verbunden zu sein, um dieselben ständig zu speisen, wenn sie in dem Einschließungsmittel untergebracht sind,
- wobei die elektrische Versorgungsschaltung einen oder mehrere männliche Steckverbinder (19) beziehungsweise weibliche Steckverbinder umfasst, die beweglich mit den entsprechenden weiblichen Steckverbindern (20) beziehungsweise männlichen Steckverbindern verbunden sind, die mit dem mindestens einen Diagnosegerät (2) und dem Schnittstellenmittel (6) verbunden sind,
- wobei die elektrische Versorgungsschaltung eine elektrische Steckdose (21) umfasst, die mit einem externen Stromnetz verbunden werden kann und mit einem oder mehreren Steckverbindern (19) der elektrischen Versorgungsschaltung (17) elektrisch verbunden ist,
wobei diese elektrische Versorgungsschaltung (17) ein Umwandlungsmittel (23) umfasst, das geeignet ist, die in der Steckdose (21) anliegende Netzspannung (V_{IN}) in eine Gleichspannung (V_{CC_1}; V_{CC_2}) umzuwandeln, die der Nennspannung des mindestens einen Diagnosegeräts (2) und/oder des Schnittstellenmittels (6) entspricht.

2. System nach Anspruch 1, **dadurch gekennzeichnet, dass** die Versorgungsschaltung (17) mindestens eine wiederaufladbare Batterie (24) umfasst, die mit den Steckverbindern (19) der elektrischen Versorgungsschaltung (17) verbunden ist und eine vorbestimmte Nenn-Ausgangsgleichspannung (V_{CC_3}) aufweist, die der Nennspannung (V_{CC_1}; V_{CC_2}) des mindestens einen Diagnosegeräts (2) und/oder des Schnittstellenmittels (6) entspricht.

3. System nach Anspruch 2, **dadurch gekennzeichnet, dass** die wiederaufladbare Batterie (24) mit der elektrischen Steckdose (21) durch ein Wiederauflademittel (25) verbunden ist, das gestaltet ist, um die Netzspannung (VIN), die in der Steckdose (21) anliegt, in eine Gleichspannung umzuwandeln, die der Nennspannung (V_{CC_3}) der wiederaufladbaren Batterie (24) entspricht.

4. System nach Anspruch 3, **dadurch gekennzeichnet, dass** die Versorgungsschaltung (17) ein Auswahlmittel (26) umfasst, das geeignet ist, selektiv die Batterie (24) mit der elektrischen Steckdose (21) oder die Batterie (24) mit dem mindestens einen Diagnosegerät (2) und/oder dem Schnittstellenmittel (6) zu verbinden.

5. System nach Anspruch 1, **dadurch gekennzeichnet, dass** das Schnittstellenmittel (6) eine Speicherschaltung (10), auf der die Anweisungen (I) einer Software (SW) gespeichert sind, und einen Prozessor (11) umfasst, der mit dieser Speicherschaltung (10) verbunden ist, um die erste Reihe von Daten (D1) nach diesen Anweisungen (I) zu verarbeiten und die zweite Reihe von Daten (D2) zu erzeugen.

6. System nach Anspruch 1, **dadurch gekennzeichnet, dass** die erste (D1) und die zweite Reihe von Daten (D2) digital sind, wobei das erste Kommunikationsmittel (5) und das zweite Kommunikationsmittel (12) aus der Gruppe ausgewählt sind, die Ethernet-Kommunikationsstandards, drahtlose Kommunikationsstandards, Bluetooth- oder ähnliche Kommunikationsstandards umfasst.

7. System nach Anspruch 6, **dadurch gekennzeichnet, dass** das mindestens eine Diagnosegerät (2) einen Umsetzer (4) umfasst, der geeignet ist, die analogen elektrischen Signale (S1), die von einem oder mehreren Sensoren (3) erzeugt werden, in die erste Reihe von digitalen Daten (D1) umzuwandeln.

## Revendications

1. Système de diagnostic portatif (1) pour la surveillance de paramètres biomédicaux (P) du corps humain, le système comprenant :
- au moins un dispositif de diagnostic (2) possédant un ou plusieurs capteurs (3) applicables au corps humain pour générer une première série de données (D1) associées à au moins un paramètre biomédical (P),
- un moyen d'interface (6) incluant un affichage (7) et un premier moyen de traitement (9), le premier moyen de traitement (9) associé audit moyen d'interface (6) étant configuré pour traiter ladite première série de données (D1) vers une seconde série de données (D2) appropriée pour être affichée dans ledit affichage (7), un premier moyen de communication (5) conçu pour transmettre ladite première série de données (D1) mesurée par au moins un dispositif de diagnostic (2) audit moyen d'interface (6),
- un second moyen de communication (12) conçu pour transmettre ladite première (D1) et/ou ladite seconde série de données (D2) dudit moyen d'interface (6) à une unité centrale de traitement (13),
- un second moyen de traitement (14) associé à ladite unité de traitement (13), configuré pour traiter ladite première (D1) et/ou ladite seconde série de données (D2) et pour les stocker dans une base de données (15),
**caractérisé en ce que**
- le système de diagnostic portatif comprend un moyen de confinement (16) conçu pour supporter ledit au moins un dispositif de diagnostic (2) et ledit moyen d'interface (6),
- un circuit d'alimentation électrique (17) fourni à l'intérieur dudit moyen de confinement (16) et conçu pour être connecté audit au moins un dispositif de diagnostic (2) et audit moyen d'interface (6) pour alimenter de façon continue ceux-ci lorsqu'ils sont logés dans le moyen de confinement,
- le circuit d'alimentation électrique comprend un ou plusieurs connecteurs mâles (19), ou respectivement des connecteurs femelles, couplés de manière mobile à des connecteurs femelles correspondants (20), ou respectivement des connecteurs mâles associés audit au moins un dispositif de diagnostic (2) et audit moyen d'interface (6),
- le circuit d'alimentation électrique comprend une prise électrique (21) pouvant être connectée à un réseau électrique externe et électriquement connectée à un ou plusieurs connecteurs (19) dudit circuit d'alimentation électrique (17),
dans lequel ledit circuit d'alimentation électrique (17) comprend un moyen de transformation (23) conçu pour transformer la tension réseau (V_{IN}) présente dans ladite prise (21) en une tension continue (V_{CC_1} ; V_{CC_2})correspondant à la tension nominale dudit au moins un dispositif de diagnostic (2) et/ou dudit moyen d'interface (6).

2. Système selon la revendication 1, **caractérisé en ce que** ledit circuit d'alimentation (17) comprend au moins une batterie rechargeable (24) connectée auxdits connecteurs (19) dudit circuit d'alimentation électrique (17) et ayant une tension de sortie CC nominale prédéterminée (VCC_3) correspondant à la tension nominale (VCC_1 ; VCC_2) dudit au moins un dispositif de diagnostic (2) et/ou dudit moyen d'interface (6).

3. Système selon la revendication 2, **caractérisé en ce que** ladite batterie rechargeable (24) est connectée à ladite prise électrique (21) par un moyen de recharge (25) conçu pour transformer la tension réseau (VIN) présente dans ladite prise (21) en une tension continue correspondant à la tension nominale (VCC_3) de ladite batterie rechargeable (24).

4. Système selon la revendication 3, **caractérisé en ce que** ledit circuit d'alimentation (17) comprend un moyen de sélection (26) conçu pour connecter sélectivement ladite batterie (24) à ladite prise électrique (21) ou ladite batterie (24) audit au moins un dispositif de diagnostic (2) et/ou audit moyen d'interface (6).

5. Système selon la revendication 1, **caractérisé en ce que** ledit moyen d'interface (6) comprend un circuit de stockage (10) sur lequel sont stockées les instructions (I) d'un logiciel (SW) et un processeur (11) connecté audit circuit de stockage (10) pour traiter ladite première série de données (D1) selon lesdites instructions (I) et générer ladite seconde série de données (D2).

6. Système selon la revendication 1, **caractérisé en ce que** ladite première (D1) et ladite seconde série de données (D2) sont numériques, ledit premier moyen de communication (5) et ledit second moyen de communication (12) étant choisis entre le groupe comprenant les normes de communication Ethernet, les normes de communication sans fil, les normes de communication Bluetooth ou similaires.

7. Système selon la revendication 6, **caractérisé en ce que** ledit au moins un dispositif de diagnostic (2) comprend un convertisseur (4) conçu pour transformer les signaux électriques analogiques (S1) générés par un ou plusieurs capteurs (3) en ladite première série de données numériques (D1).
